# EUROPEAN PATENT SPECIFICATION

(11) **EP 1 907 543 B1**
(45) Date of publication and mention of the grant of the patent: **25.09.2013**
(21) Application number: 06765969.8
(22) Date of filing: 30.06.2006
(51) Int. Cl.: C12N 15/09

(54) **PRODUCTION OF RECOMBINANT PRODUCTS USING CAPILLARY MEMBRANES**
HERSTELLUNG VON REKOMBINANTEN PRODUKTEN UNTER VERWENDUNG VON KAPILLARMEMBRANEN
PRODUCTION DE PRODUITS RECOMBINES UTILISANT DES MEMBRANES CAPILLAIRES

(30) Priority: 30.06.2005 ZA 200505315
(43) Date of publication of application: 09.04.2008
(73) Proprietor: Quorus Biotech (Proprietary) Limited, Mowbray 7700 (ZA)
(72) Inventor: LEUKES, Winston, Daniel, 8005 Sea Point (ZA); FRASER, Sheena, Janet, 8005 Sea Point (ZA)
(74) Representative: Jones, Helen M.M.
(86) International application number: PCT/IB2006/052204
(87) International publication number: WO 2007/004172

(56) References cited:
- INLOES DOUGLAS S ET AL: "HOLLOW-FIBER MEMBRANE BIOREACTORS USING IMMOBILIZED E. COLI FOR PROTEIN SYNTHESIS" BIOTECHNOL BIOENG NOV 1983, vol. 25, no. 11, November 1983 (1983-11), pages 2653-2681, XP002406100
- RAJAGOPALAN VENKATADRI ET AL: "CULTIVATION OF PHANEROCHAETE CHRYSOSPORIUM AND PRODUCTION OF LIGNIN PEROXIDASE IN NOVEL BIOFILM REACTOR SYSTEMS: HOLLOW FIBER REACTOR AND SILICONE MEMBRANE REACTOR" WATER RESEARCH, ELSEVIER, AMSTERDAM, NL, vol. 27, no. 4, 1 April 1993 (1993-04-01), pages 591-596, XP000356361 ISSN: 0043-1354
- CANOVAS ET AL: "Factors affecting the biotransformation of trimethylammonium compounds into l-carnitine by Escherichia coli" BIOCHEMICAL ENGINEERING JOURNAL, ELSEVIER, AMSTERDAM, NL, vol. 26, no. 2-3, 15 November 2005 (2005-11-15), pages 145-154, XP005097671 ISSN: 1369-703X

## Description

### FIELD OF INVENTION

THIS INVENTION relates to the production of recombinant products. In particular, it relates to a method of producing recombinant products under aerobic conditions, and to apparatus for producing such recombinant products under aerobic conditions.

### PRIOR ART

The application of hollow fiber membrane cartridges to microbial cultivations has been described previously. The principle aim of using membrane cartridges for cell cultivation is to enable continuous high cell density cultivation for improved productivity. It is however the configuration, operation and application of these membrane cartridges that ultimately distinguish different cell cultivation process from each another. For example, Canovas *et al.,* (2005) use a standard reactor vessel connected to a cross-flow membrane filtration cartridge for recycling and concentration of *E.coli* within the reactor vessel. In contrast, Inloes *et al.,* (1983) sought to use the membrane cartridge itself as the cell cultivation vessel through the entrapment of actively growing *E.coli* within the macroporous membrane matrix, with nutrients replenished by diffusion from the membrane lumen; while Leukes et al., (EP 0761608, published 12 March 1997) immobilized the filamentous fungus *P.chrysosporium* as a biofilm on the outer membrane surface, with nutrients supplied to immobilized cells from the lumen by convective flow through the membrane wall to the biofilm of cells.

### BACKGROUND TO INVENTION

Recombinant products, including but not limited to enzymes, hormones, antibodies, antibody fragments, virus like particles, peptides, DNA and RNA fragments are produced by a variety of microbial expression systems. Products may also include chemical products resulting from expression of recombinant synthetic enzymes.

The market for recombinant products for the pharmaceutical as well as other industries has increased in recent years. Commercial products include both recombinant products expressed in recombinant hosts as well as chemicals produced by recombinant biosynthetic enzymes.

The efficient production of such recombinant products typically relies on the use of efficient expression hosts as well as good bioprocess technology. Expression hosts include a number of bacterial species of which *Escherichia coli* is most prominent, as well as fungi, protozoa, plant, insect and mammalian cells. Some of the products are intracellular, but it is preferred that products be excreted into the medium for easy purification. Most expression systems are aerobic, requiring considerable input of energy to increase oxygen mass transfer in submerged culture bioreactors. Most bioprocess technology is focused on obtaining very high cell concentrations in the bioreactor, typically by fed-batch culture mode. Production hosts are either engineered to produce recombinant products of interest constitutively or once sufficient biomass is present the production hosts are induced to express recombinant products of interest. This induction is typically a molecule that is added at the appropriate time or is a metabolite produced by the production host as a metabolic waste product or a secondary metabolite.

The strategy of inducing recombinant protein production once the culture is in the stationary phase is typical since:
1) It allows for the production of products that are toxic to the producing organism.
2) Volumetric productivity is improved if the biomass concentration in the reactor is very high.
3) Precursors for the formation of the product are typically primary metabolites, which accumulate during the primary growth phase.

In submerged batch culture it is often difficult to time the addition of recombinant protein inducers optimally so that inducers are added when the culture reaches stationary phase and not too soon or too late. It is particularly difficult when multiple cultures are run in parallel under different conditions during optimization experiments.

Although most recombinant protein expression hosts are aerobic organisms, high density culture is limited because of oxygen mass transfer limitations and toxicity or solubility constraints of nutrients supplied in growth medium. Factors that affect the volumetric productivity of bioreactors for recombinant protein production include: low shear environment, high biomass concentration, extended production phase, good nutrient mass transfer.

Typically, in high density culture oxygen and nutrient mass transfer is influenced by culture morphology and/or rheology. In submerged culture, expression of recombinant products in filamentous organisms such as *Aspergillus* are particularly affected by morphological differentiation and pellet formation and productivity has been enhanced through immobilization of the recombinant organism. Immobilisation not only limits shear stress and reduces broth viscosity, improving oxygen supply and nutrient mass transfer, but was also shown to inhibit extracellular protease production in *Aspergillus niger* thereby limiting degradation of recombinant proteins produced in this organism (Wang *et al.* 2005). In most immobilized systems oxygen mass transfer limitations remain a rate limiting step in productivity.

Inloes et al (1983) sought to improve recombinant protein production through the entrapment of actively growing *E.coli* within the macroporous matrix of hollow fiber membrane wall. This solution resulted in a low sheer environment with high biomass concentration; however nutrient mass transfer was limited to diffusion from the membrane lumen to entrapped cells, while entrapment of actively growing cells proved problematic as the membrane wall was ruptured and the nutrient feed became contaminated.

The present invention overcomes the problems encountered by Inloes *et al.,* (1983) by immobilizing the microorganism on the membrane surface as a biofilm of cells and controlling the rate of nutrient delivery to the microorganisms such that a portion of immobilized cells is maintained as actively growing cells while a further portion enters into and is maintained in the stationary phase of growth as described by Leukes et al., (EP 0761608, published 12 March 1997) such that stationary phase cells can thereby be induced to produce recombinant products that can be continuously harvested from these stationary phase cells.

Any reference herein to a "microorganism" must be interpreted to mean a reference to organisms which are members of the bacteria, fungi, protozoa and includes plant or mammalian cells that have been engineered to produce recombinant products under aerobic conditions.

Any reference herein to a "stationary phase" must be interpreted to mean a period of limited growth or growth and death substantially in equilibrium which typically follows immediately after the primary growth phase in batch or continuous cultivation of microorganisms.

Any reference herein to "recombinant protein" must be interpreted as synonymous with "recombinant product" and to mean any product or the product thereof if it is a biosynthetic product, homologous or heterologous to the expression host, the production of which may be induced by some mechanism at the onset of - or during the stationary phase of a typical batch process. The product can either be secreted by the expression host into the extracellular environment or retained intracellularly. Recombinant products are not limited to proteins.

Any reference herein to a "nutrient solution" must be interpreted to mean a liquid solution containing one or more nutrients required for microorganism growth and which includes at least one nutrient which is growth limiting. The nutrient solution may also carry an inducer required for initiating the expression of recombinant products.

Any reference herein to an "inducer" must be interpreted to mean a biological or synthetic chemical included in the nutrient solution or a biosynthetic product produced by the immobilised expression host during growth and used as a means of regulating the production of recombinant products or proteins under the control of a relevant promoter regulating expression of the said recombinant products or proteins.

Any reference herein to "permeate" must be interpreted to mean spent nutrient solution or product stream that has passed through a substrate and biofilm and may carry secreted product, metabolic waste products and/or the remainder of nutrients not metabolised by the microorganism during passage of nutrient solution through the biofilm.

Any reference herein to "aerated conditions" must be interpreted to mean any cultivation condition in which a gas, which contains oxygen, is passed through a bioreactor across a biofilm surface, and which supplies microorganisms with oxygen for growth.

### SUMMARY OF INVENTION

According to one aspect of the invention, there is provided a method of producing at least one recombinant product under aerated conditions, which method includes:
providing a porous substrate having a first side and a second side and which has a biofilm of microorganisms attached to the first side thereof, the substrate being configured to allow passage of a nutrient solution therethrough and to prevent the passage of microorganism cells therethrough; and
causing a nutrient solution to flow through the substrate and the biofilm in a direction from the second side thereof to the first side thereof under aerated conditions, at a rate which is sufficiently low for a nutrient gradient to be established across the biofilm wherein the nutrient concentration is relatively higher closer to the substrate and sufficiently high to support primary growth of the microorganisms and wherein the nutrient concentration is relatively lower further from the substrate and sufficiently low to enter and sustain a stationary phase of the microorganisms, and the microorganisms being induced to produce recombinant product.

Induction may be caused in various ways such as by nutrient depletion, addition of an inducer molecule, accumulation of a primary metabolic product or secondary metabolite produced under nutrient limiting conditions, auto-induction, or the like. In other words, the method may use derepression and/or induction of recombinant gene expression. In other words the induction may occur through the addition of an inducer molecule to the nutrient solution or through the accumulation of a primary metabolic product or secondary metabolite produced under nutrient limiting conditions.

The induction may occur through the addition of an inducer molecule to the nutrient solution such that the flow of the nutrient solution from the second side through the substrate and the biofilm to the first side carries said inducer to the microorganism thereby to induce production of at least one recombinant protein or product by the microorganisms. Induction may also occur through the accumulation of primary or secondary metabolites within the biofilm such that as they are carried through the biofilm in the nutrient solution resulting in a concentration gradient wherein the inducer concentration is lower closer to the substrate and higher further from the substrate and sufficiently high to induce expression of recombinant products.

The recombinant product may be collected in the permeate from the first side if secreted by the microorganisms or may be harvested from the biofilm if retained intracellularly by the microorganisms.

The inducer may be any suitable inducer such as isopropyl b-D-thiogalacto side (IPTG) (0.1-1 mM), methanol (0.5-1.5%) or organic acids produced by the microorganism that interact with the specific promoter with which the recombinant expression system is engineered. Concentration of inducer may be varied in order to optimize the expression of soluble, correctly folded protein products.

The method may include contacting the biofilm with a gas. The gas may be blown over an outside surface of the biofilm thereby to carry away spores and dead cells of the microorganisms as well as to remove spent nutrient solution which may contain secreted product. The gas may provide oxygen for metabolism. The gas may be air.

The microorganisms may be a substantially pure culture of a strain of a microorganism or a mixed culture of different microorganisms. The microorganisms may be selected from the group consisting of *Aspergillus* sp., *Trichoderma* sp., *Mucor* sp., *Pichia* sp., *Fusarium* sp., *Neurospora* sp., *Penicillium* sp., *Streptomyces* sp., *Chrysosporium lucknowense, Mortierella alpinis,*

The method of producing the recombinant product may be carried out at a temperature within the range of growth and product formation of the microorganisms. The method of producing the recombinant product may be carried out at a temperature of 2-109 °C, preferably 20 - 40 °C, e.g. 30 °C.

The method of producing the recombinant product may be carried out at an initial pH within the range of growth and product formation of the microorganisms. The method of producing the recombinant product may be carried out at an initial pH of 1-13, preferably 4 - 8, e.g. 7.

The method of producing the recombinant product may be carried out for a period for as long as the biofilm or culture remains viable and productive and not too dense so as to prevent product flow through the membrane. The method of producing the recombinant product may be carried out for a period of 2 - 60 days, preferably 2 - 30 days, e.g. 25 days.

The recombinant product may be produced intracellularly or extracellularly by the microorganisms. The recombinant product may be collected from the first side if secreted by the microorganisms or may be harvested from the biofilm if retained intracellularly by the microorganisms. Intracellular metabolites may be retained within the cytoplasm or secreted to the periplasmic space and extracted using cell lysis or cell permeabilisation procedures such as osmotic shock.

Nutrient solution may be passed through the biofilm and substrate at a flow rate that is sufficient to sustain and immobilise the microorganism while maintaining a nutrient gradient across the biofilm. Flow rates may differ depending on the nutrient content of the nutrient solution and or the type of microorganism cultured. Flow rates may range from 0.001 - 10 volumes nutrient solution per reactor volume per hour.

Biofilm thickness may depend on the nutrient content of the nutrient solution and the type of microorganism cultured. The biofilm may have a thickness of 0.1 - 10 mm and should be limited such that optimal mass transfer of oxygen between humidified air and the biofilm is maintained within a suitable range for the biofilm or culture to remain viable and productive.

Also discussed herein is an apparatus for producing a recombinant product under aerated conditions, which includes:
at least one porous substrate having a first side and a second side; and
a feeding arrangement for supplying a nutrient solution under aerated conditions into contact with the microorganisms which become attached to and form, in use, a biofilm on the first side of the substrate, the feeding arrangement being operable to cause the nutrient solution to flow through the substrate and the biofilm in a direction from the second side thereof to the first side thereof at a rate sufficiently low for a nutrient gradient having to be established across the biofilm wherein the nutrient concentration is higher closer to the substrate and sufficiently high to support primary growth of the microorganisms and wherein the nutrient concentration is lower further from the substrate and sufficiently low to enter and sustain a stationary phase of the microorganisms, with the microorganisms being induced to produce recombinant product. Induction may also occur through the accumulation of primary or secondary metabolites within the biofilm such that as they are carried through the biofilm in the nutrient solution resulting in a concentration gradient wherein the inducer concentration is lower closer to the substrate and higher further from the substrate and sufficiently high to induce expression of at least one recombinant product by the organism.

The feeding arrangement may include at least one of a mechanical source such as a pump and a pneumatic source such as compressed air, for feeding the nutrient solution into contact with the substrate at the second side and causing the nutrient solution to flow through the substrate and the biofilm to the first side.

The apparatus includes a pneumatic source such as compressed air supplied at the first side which may flow across and be directed into contact with the outside of the biofilm. The gas should include oxygen for growth and metabolism and may also be used to carry away spores and dead cells of the microorganism and to remove permeate which may contain secreted product.

The apparatus may include product collection means for collecting the gas and the permeate, which may contain product, from the first side of the biofilm. The product collection means may also include an outlet including a backpressure creating means such as, but not limited to, a mechanical restriction, which may be used to regulate the pressure of the gas at the first side thereby altering oxygen transfer of the gas to the biofilm under the resultant applied backpressure.

The substrate may comprise a porous membrane having apertures sufficiently large to permit the flow of the nutrient solution therethrough, but which apertures are sufficiently small to prevent passage or growth of microorganism cells therethrough. The exact configuration of the membrane may vary greatly. In particular embodiments, the membrane may be in the form of a hollow-fibre membrane, a capillary membrane or may have a tubular or flat sheet configuration.

The apparatus may include a housing for the substrate, the housing being spatially separate from the substrate. The housing may include an inlet for the nutrient solution, an inlet for the gas solution and an outlet for discharging permeate from the housing.

Unless otherwise defined, all technical and scientific terms used herein have the same meaning as commonly understood by one of ordinary skill in the art to which this invention pertains. In case of conflict, the present document, including definitions, will override terms commonly understood by one of ordinary skill in the art. Preferred methods and apparatus are described below, although methods and apparatus similar or equivalent to those described herein can be used in the practice or testing of the present invention. All publications, patent applications, patents and other references mentioned herein are incorporated by reference in their entirety. The methods, apparatus and examples disclosed herein are for illustrative purposes only and not intended to be limiting.

### BRIEF DESCRIPTION OF DRAWINGS

Further features of the invention are described hereinafter by way of a non-limiting example of the invention, with reference to and as illustrated in the accompanying diagrammatic drawings. In the drawings;
**Figure 1** shows a schematic side elevation of an apparatus in accordance with the method of the invention;
**Figure 2** shows a schematic sectional plan view of the porous substrate and biofilm, sectioned I, II and III, supplied with nutrients from the second side to the first side and oxygen supplied at the first side such that a nutrient gradient is established across the biofilm that is highest at the second side and lowest at the first side, and an oxygen gradient is established that is highest at the first side and lowest at the second side;
**Figure 3** shows a schematic view of a plurality of single-fibre membrane bioreactors forming a scaled-up embodiment of the apparatus used in the invention.

### BRIEF DESCRIPTION OF THE PREFERRED EMBODIMENT

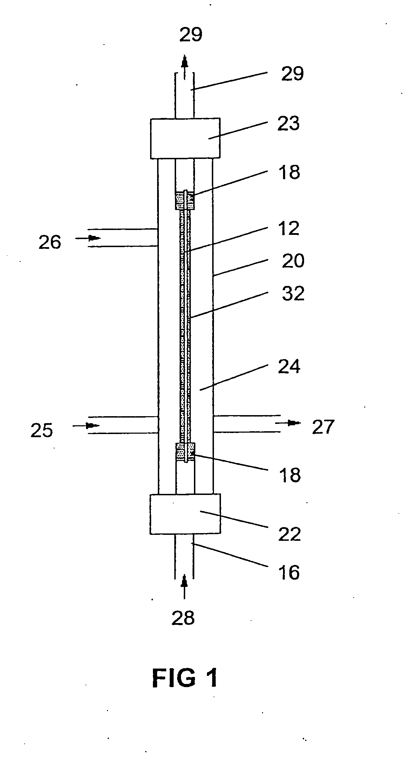

With reference to Figures 1 to 2 of the drawings, an apparatus for producing a recombinant protein under aerated conditions, in accordance with the method of the invention, is designated generally by the reference numeral 10. The apparatus is in the form of a bioreactor 10 shown in Figure 1 of the drawings at a laboratory scale, however, it will be appreciated that the principles embodied in the bioreactor 10 can be applied to a scaled-up or commercial embodiment.

The bioreactor 10 includes a ceramic hollow fibre capillary membrane 12 with ends of the membrane being potted into plastic inserts 14, 16 with epoxy 18. The housing or reactor shell 20 of glass is arranged coaxially with the capillary membrane 12 and is provided with end caps 22 and 23 which thread onto the glass housing 20. The housing 20 includes an air inlet 26 for introducing a oxygen into the space 24 between the capillary membrane 12 and the housing 20, inoculation inlet 25 for introducing an inoculum into the space 24 between the housing 20 for attachment to the membrane 12. The housing further includes a permeate and air outlet 27 for discharging the feed solution and flowing air from the housing.

In use, a biofilm 32 is established on an external surface 30 of the capillary membrane 12. This is achieved by introducing a spore or vegetative inoculum of the desired microorganism into the space 24 between the membrane surface 30 and the glass housing 20 at the first side and filtering the inoculum through the capillary membrane 12 from the external surface 30 towards the membrane lumen 34 at the second side, and draining any permeate out of the lumen 34 and through the outlet 29. The lumen 34 is thus in flow communication with the outlet 29. The inoculum is thus immobilised on the membrane surface 30. The membrane 12 has a relatively thin, porous skin 36 on the inside and a relatively large, porous, externally unskinned void structure 38 radiating outwardly from the skin 36. Typically, the membrane 12 has an outside diameter of about 2mm.

During operation an appropriate nutrient solution for the microorganism is introduced into the housing via the medium inlet 28. The nutrient solution is caused to flow through the membrane 12 in a direction from the second side or lumen of the membrane 34 to the first side or outer surface thereof 30. Air is displaced from the membrane lumen 34 or second side through a medium outlet 29 which is closed off during operation to facilitate permeation of the nutrient feed from the lumen side, through the capillary membrane wall 36 and 38 to the space 24 between the membrane surface 30 and the glass housing 20.

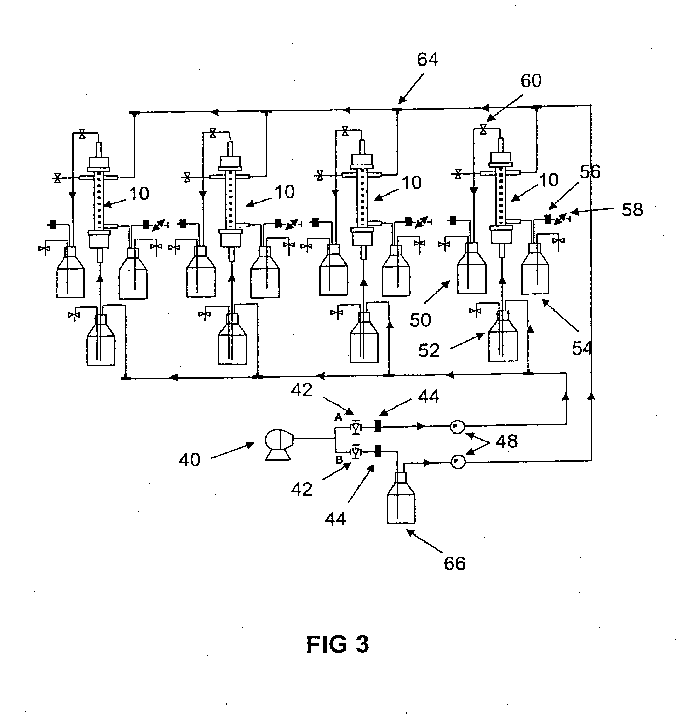

In Figure 3, a plurality of bioreactors 10 are illustrated. A compressed air supply is indicated by reference numeral 40 is connected via two regulator valves 42 to two 0.22m filters 44. A humidification vessel 46 is provided and pressure is measured through pressure gauges 48. Priming vessels 50 and medium supply vessels 52 are shown connected to the bioreactors 10. Permeate collection vessels 54 are connected to vent filters 56 and flow regulators 58.

With reference to Figure 3, the line to the permeate collection vessel 54 is mechanically sealed by means of a clamp 60 before the microorganism is introduced directly into the space between the membrane surface and the glass housing of each bioreactor 10 at an inoculation inlet 62, such that the space between the membrane surface and glass housing is filled with inoculum. The inoculation inlet 62 is then mechanically sealed with the clamp 60 and the inoculum is immobilised onto the membrane surface under pressure, supplied from the compressor 40 through line B. The pressure of compressed air is controlled by the pressure regulator valve 42 and monitored using the pressure guage 48. T-pieces 64 distribute the pressure evenly between the manifolded bioreactors 10. The pressurised air from line B is sterilised by passing the gas through the 0.22 µm filter 44 and then humidified by bubbling the air stream through sterile distilled water in a humidification vessel 66 prior to entering the bioreactor 10, in order to prevent desiccation of the microorganism and to improve the oxygen transfer rate between the air stream and the biofilm. The inoculation filtrate is collected from the lumen side into the priming vessel 50. Once complete the line to permeate collection vessel 54 is opened and the air pressure and the air flow rate from line B is set at the air outlet of the permeate collection vessel 54 using the flow regulator 58.

During operation nutrient solution is supplied from the medium supply vessel 52 under pressure supplied by from the compressor 40 through line A. The pressure of compressed air is controlled by the pressure regulator valve 42 and monitored using a pressure guage 48. T-pieces 64 distribute the pressure evenly between the manifolded bioreactors 10. The pressurised air from line A is sterilised by passing the gas through the 0.22 µm filter 44. The nutrient solution is fed pneumatically from the medium supply vessel 52 into the lumen of the capillary membrane, displacing air from the lumen into the priming vessel 50. During operation the line to the priming vessel 50 is mechanically sealed with the clamps 60. The rate at which nutrient solution is supplied from the membrane lumen at the second side to the biofilm immobilised on the membrane surface at the first side is determined by the pressure differential between the compressed air supply of line B and the compressed air supply of line A. Permeate can be removed at intervals from the permeate collection vessel 54 through a sampling port 68.

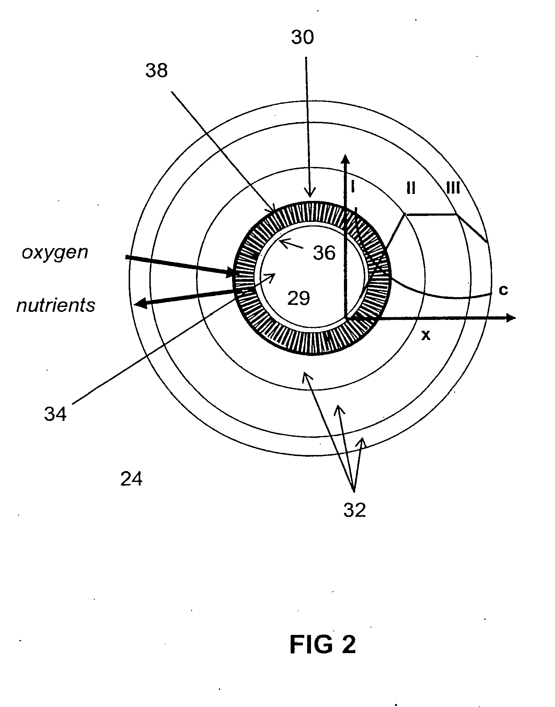

Fundamental to the production of recombinant proteins is the regulation of gene expression by molecules that either repress or induce gene expression by acting on a promoter element controlling expression of the foreign gene. In addition to better known inducers such as I PTG and methanol, nutrients such as carbon or nitrogen may serve to either down-regulate (repress) or up-regulate (induce) the expression of a recombinant product. Through nutrient starvation the promoter element may be de-repressed and or induced, thus initiating gene expression and recombinant protein production. This is achieved with the membrane-immobilised biofilm bioreactor 10 by the production of radial nutrient concentration gradients through the biofilm 32. As a result, the nutrient concentration at the membrane / biofilm interface is high, whereas the nutrient concentration at the exposed outer surface of the biofilm 32 is low. In Figure 2 of the drawings, for the purposes of illustration, a graph depicts the nutrient concentration level C (y axis) vs. distance from the membrane surface (x axis) while nutrient concentration zones from high to low are designated by the reference numbers I to III, respectively. In zone I the nutrient concentration is sufficiently high to support primary growth of the microorganisms, in zone II the nutrient concentration is sufficiently low to cause nutrient starvation which stresses the microorganisms thereby to induce and sustain a stationary phase of the microorganisms, while in zone III the nutrient concentration may be depleted resulting in cell death and lysis.

Due to the toxicity of many recombinant products and the high cell densities required for increased productivity most expression systems have been engineered to produce recombinant products during stationary phase growth through either auto-induction or the addition of an inducer during stationary phase growth. Auto-induction may result from
1) the nutrient gradients described herein; or
2) as the concentration of metabolic waste product increases as the nutrient solution perfuses the biofilm, which may also induce product formation in cultures where the accumulation of metabolic waste induces product formation;
3) this could also result in the production of a secondary metabolite which induces the production of a recombinant product. It will be appreciated that after the establishment of the nutrient gradient, product formation may also be induced by altered pH as a result of organic acid accumulation.

In this invention optimal conditions for expression may be maintained while limiting exposure of the recombinant host to toxic products. When operated the bioreactor allows for extended production of recombinant proteins and as the biomass is retained, allows for the recovery of a relatively high purity, biomass free product. In addition, degradation and decay of recombinant products by proteolytic enzymes is reduced through the continuous removal of product.

In a recombinant host where expression is auto-regulated by nutrient conditions or metabolic products produced by the host organism itself, recombinant protein production may be maintained in zone II e.g Xylanase production in *Aspergillus niger* under the control of the glyceraldehydes-6-phosphate dehydrogenase (gpd) promoter (Rose and van Zyl, 2002).

In a recombinant host where expression is regulated by a synthetic or biosynthetic chemical, recombinant protein production may be induced throughout the biofilm in the absence of an inhibitor or recombinant protein production may be maintained in zone II where such an inhibitor may be metabolized by the microorganism e.g. Recombinant production in *Pichia pastoris* under the control of the AOX promoter is induced by methanol under carbon limiting conditions. In this expression system albumin is a secreted product and β-galactosidase is an intracellular product.

It will be appreciated that the regulation of recombinant protein production is host and expression system specific and that the means by which recombinant protein production is achieved within the immobilised biofilm may differ while still incorporating the essential features of the nutrient, metabolite and/or inducer gradients described herein.

It will also be appreciated that the exact configuration of the bioreactor and of the fluid flow regime may vary greatly while still incorporating the essential features defined hereinabove.

Methods of secondary metabolite production are described in patents US5945002 and EP-A-1907542

### EXAMPLE 1

### Aspergillus niger expression system

*Aspergillus niger* has been used routinely as a fungal expression host using a variety of expression vectors. In this example *A. niger* engineered to constitutively express the *Trichoderma reesei* β-1,4-xylanase gene *(xyn2)* under the transcriptional control of the glyceraldehydes-6-phosphate dehydrogenase *(gpd)* promotor from *A. niger* (Rose and van Zyle, 2002) is used to demonstrate the continuous production of recombinant xylanse by this stable, high yielding transformant using the apparatus described herein.

### Strain and Cultivation Conditions

Recombinant strain *Aspergillus niger* D15 containing the xylanase II (xyn2) gene of *Trichoderma reesei* integrated into its fungal genome was obtained from Prof. W.H. van Zyl, Dept. of Microbiology, University of Stellenbosch, South Africa (Rose and van Zyl, 2002).

The xylanase producing *Aspergillus* was cultivated in 20% (v/v) mollasses, 1 x defined medium (Punt and van den Hondel, 1992), 2x defined medium (Punt and van den Hondel, 1992) or minimal medium (Record *et al.* 2002).

### Bioreactor Setup

Single fibre membrane bioreactors were configured as illustrated in Figure 3. Four banks of four bioreactor modules were operated, each bank under different nutrient conditions. Bioreactors were autoclaved and setup for aerobic operation according to standard operating procedures (SOPs). Sterile growth medium was aseptically dispensed into each of the medium supply vessels prior to starting the experiment.

### Inoculation

All bioreactor were inoculated with 1 ml spore suspension generated by resuspending spores from a single agar plate in 20 ml sterile distilled water. Inoculum was aseptically injected directly into the extracapillary space (ECS) i.e. between the membrane surface and the glass housing of each bioreactor. Immobilisation of inoculum on the outer surface of capillary membranes was completed by reverse flow, according to SOPs.

### Operation

Bioreactors were operated under aerobic conditions according to SOPs. Oxygen in the form of 0.22 µm filtered, humidified air was supplied from a compressor using a pressure regulator valve and monitored using a pressure gauge. Hoffman clamps were used at the air outlet of each bioreactor to regulate airflow and create backpressure. The air pressure within the ECS i.e. the space between the membrane surface and the glass housing at the first side was monitored using a pressure gauge and maintained at 60 kPa and the airflow through the bioreactor was set to 1 vol air per minute per reactor volume using a rotameter.

Growth medium was supplied to each bioreactor separate medium supply vessels using 0.22 µm filtered compressed air using a pressure regulator valve and monitored using a pressure gauge. Growth medium was fed to the biofilm from the lumen or second side of capillary membranes, through the membrane wall to the first side or outer surface of the capillary membrane. Medium flow rate from the second side to the first side was manually regulated according by maintaining a positive pressure differential set across the membrane surface from lumen or second side to outer membrane surface or first side of the bioreactor, thus controlling the rate of nutrient feed (flux) to the biofilm. Increased biofilm growth creates greater resistance to flow and the pressures should be adjusted accordingly to maintain constant flux. Under optimal flux conditions the biofilm thickness and flux are maintained substantially in equilibrium facilitating the nutrient gradients required for differentiation of the microorganism and continuous recombinant protein production.

Permeate was collected in permeate collection vessels and sampled daily for further analysis. The rate of permeate accumulation and pH was monitored daily. Samples were stored at -20°C for further analysis.

### Biofilm development

Using different growth medium different biofilm thickness, differentiation and productivity were observed for the *A. niger* D15 transformant.

Biofilm growth developed rapidly in the minimal medium (Record *et al.* 2002) showing even, black pigmented and heavily sporulating growth as early as day 3.

Growth on 1 x and 2x defined medium (Punt and van den Hondel, 1992) was similar, with slightly greater biofilm thickness when using 2x growth medium. Growth was slower than in defined medium and did not demonstrate the same dark pigmentation or sporulation. Biofilm pigmentation was mottled cream to light and dark brown, darkening from day 10 and sporulating from day 15. Growth in molasses was poor, showing only effuse biofilm growth with dark pigmentation and sporulation by day 14.

With reference to Table 1, bioreactors were harvested after 25 days and the dry cell weights calculated for each bioreactor. Significantly higher biomass was obtained using 2x defined medium. The amount of xylanase produced by bioreactors over a 25 day period was strongly correlated with biomass yield (r² = 0.92).

**Table1 : Average biomass produced in bioreactors (n = 2) cultured with different growth medium**

| | Dry cell weight (g) | | Units xylanase per g biomass | |
|---|---|---|---|---|
| | Mean | SD | Mean | SD |
| Minimal medium | 0.53 | 0.04 | 4843 | 3103 |
| 2x defined medium | 1.71 | 0.08 | 42071 | 1095 |
| 20 % molasses | 0.12 | 0.05 | 8282 | 3232 |
| 1x defined medium | 0.66 | 0.05 | 5647 | 94 |

### Productivity

Enzyme activity was quantified using Megazyme's azo-xylan (birchwood) xylanase activity assay. The procedure was performed in sodium phosphate buffer, pH 6.0 at 55°C. The pH and incubation temperatures used were previously described as optimal for recombinant xyn2 produced in *Aspergillus niger* D15 (Rose and van Zyl, 1992). Separate controls (blanks) were prepared for each sample due to differences in medium composition between bioreactors. One unit of enzyme activity is defined as the amount of enzyme required to release one µmole of D-xylose reducing-sugar equivalents from arabinozylan, per minute.

With reference to Table 2, defined growth medium showed greater yields of xylanase production, with 2x defined medium doubling the volumetric productivity of bioreactors in comparison to 1 x defined medium. This was correlated with the increased amount of biomass produced when using a more concentrated growth medium (as above). Bioreactors cultured using minimal medium showed were marginally less productive than those cultured with 1 x defined medium, while 20 % molasses supported poor growth and xylanase production in bioreactors.

**Table 2: Xylanase production by bioreactors.**

| Bioreactor | Xylanase (Units/l) | | | Space Time Yield (Units/h per I. reactor volume) | | | Xylanase (Units) |
|---|---|---|---|---|---|---|---|
| | Max. | mean | SD | Max. | mean | SD | Per 25 day period |
| 1 | 13.53 | 9.67 | 3.43 | 2.20 | 0.72 | 0.75 | 3498 |
| 2 | 5.55 | 7.81 | 3.65 | 1.09 | 0.31 | 0.34 | 1470 |
| 3 | 18.63 | 10.74 | 4.43 | 2.87 | 0.75 | 0.81 | 3621 |
| 4 | 17.86 | 10.02 | 4.35 | 4.30 | 0.90 | 1.18 | 4311 |
| 5 | 54.29 | 17.98 | 14.98 | 6.10 | 1.38 | 1.63 | 7042 |
| 6 | 33.88 | 16.83 | 12.14 | 8.68 | 1.63 | 2.20 | 8250 |
| 7 | 27.28 | 12.79 | 6.85 | 2.36 | 1.02 | 0.74 | 6059 |
| 8 | 31.98 | 12.76 | 7.53 | 2.92 | 1.17 | 0.73 | 6940 |
| 9 | 3.64 | 2.26 | 1.13 | 0.55 | 0.21 | 0.14 | 1135 |
| 10 | 4.40 | 2.31 | 1.24 | 0.44 | 0.17 | 0.12 | 898 |
| 11 | 3.23 | 2.06 | 1.02 | 0.30 | 0.18 | 0.13 | 965 |
| 12 | 4.30 | 1.97 | 1.47 | 0.58 | 0.14 | 0.15 | 713 |
| 13 | 21.99 | 12.38 | 6.00 | 1.11 | 0.65 | 0.36 | 3493 |
| 14 | 30.20 | 15.70 | 8.39 | 1.44 | 0.72 | 0.43 | 3993 |
| 15 | 22.76 | 13.51 | 6.19 | 1.28 | 0.74 | 0.42 | 4005 |
| 16 | 23.51 | 15.95 | 7.74 | 1.78 | 0.81 | 0.46 | 4231 |

### References:

1. Wang, L., Ridgway, D., Gu, T. and Moo-Young M. (2005) Bioprocessing strategies to improve heterologous protein production in filamentous fungal fermentations. Biotech. Advances 23, pp. 115-129
2. Rose, S.H and van Zyl W.H. (2002) Constitutive expression of the Trichoderma reesei beta-1,4-xylanase gene (xyn2) and the beta-1,4-endoglucanase gene (egl) in Aspergillus niger in molasses and defined glucose media. Appl. Microbiol. Biotechnol. 58, pp. 115-129.

## Claims

1. A method of producing at least one recombinant product under aerated conditions, which method includes:
providing a porous substrate having a first side and a second side and which has a
biofilm of microorganisms attached to the first side thereof, the substrate being configured to allow passage of a nutrient solution therethrough and to prevent the passage of microorganism cells therethrough; and
causing a nutrient solution to flow through the substrate and the biofilm in a direction from the second side thereof to the first side thereof under aerated conditions, wherein the flow rate of the nutrient solution from the second side to the first side is 0.001 - 10 volumes nutrient solution per bioreactor volume per hour, thereby providing a nutrient gradient having a concentration differential across the biofilm wherein the nutrient concentration is higher closer to the substrate thereby to support primary growth of the microorganisms and wherein the nutrient concentration is lower further from the substrate thereby to cause the microorganisms to enter and sustain a stationary phase, and the microorganisms being induced to produce recombinant product.

2. A method of claim 1, wherein the microorganisms are selected from the group consisting of *Aspergillus* sp., *Trichoderma* sp., *Mucor* sp., *Pichia* sp., *Fusarium* sp., *Neurospora* sp., *Penicillium* sp., *Streptomyces* sp., *Chrysosporium lucknowense, Mortierella alpinis.*

3. A method of either claim 1 or 2, wherein the method is carried out for a period of 2 - 60 days.

4. A method of any one of claims 1 to 3, wherein the biofilm has a thickness of 0.1 - 10 mm.

5. A method of any one of claims 1 to 4, which includes adding an inducer molecule to the nutrient solution such that the flow rate of the nutrient solution from the second side through the substrate and the biofilm to the first side carries said inducer to the microorganism thereby to induce production of at least one recombinant product by the microorganisms and wherein the inducer is optionally a primary or secondary metabolite that accumulates within the biofilm, such that as the inducer is carried through the biofilm in the nutrient solution this results in a concentration gradient wherein the inducer concentration is lower closer to the substrate and higher further from the substrate thereby to induce expression of at least one recombinant product by the microorganism.

6. A method of claim 7, wherein the inducer is selected from at least one of isopropyl b-D-thiogalacto-side (IPTG) (0.1-1 mM), methanol (0.5-1.5%) and organic acids produced by the microorganism.

7. A method of any one of claims 1 to 6, wherein the recombinant product is produced intracellularly by the microorganisms.

8. A method of any one of claims 1 to 6, wherein the recombinant product is produced extracellularly by the microorganisms.

9. A method of any one of claims 1 to 8, which includes contacting the biofilm with a gas.

## Patentansprüche

1. Verfahren zur Herstellung wenigstens eines rekombinanten Produkts unter Belüftungsbedingungen, wobei man bei dem Verfahren:
ein poröses Substrat mit einer ersten Seite und einer zweiten Seite bereitstellt, an dessen erster Seite ein Biofilm von Mikroorganismen angebracht ist, wobei das Substrat so konfiguriert ist, dass es die Passage einer Nährlösung durch es hindurch gestattet und die Passage von Mikroorganismuszellen durch es hindurch verhindert; und
eine Nährlösung dazu bringt, durch das Substrat und den Biofilm in einer Richtung von der zweiten Seite davon zu der ersten Seite davon unter Belüftungsbedingungen zu fließen, wobei die Fließgeschwindigkeit der Nährlösung von der zweiten Seite zu der ersten Seite 0,001 - 10 Volumen Nährlösung pro Bioreaktorvolumen pro Stunde beträgt, wodurch ein Nährstoffgradient mit einem Konzentrationsunterschied über den Biofilm hinweg bereitgestellt wird, wobei die Nährstoffkonzentration näher zum Substrat hin höher ist, um dadurch die Primärkultur der Mikroorganismen zu unterstützen, und wobei die Nährstoffkonzentration weiter vom Substrat entfernt niedriger ist, um dadurch die Mikroorganismen dazu zu bringen, in eine stationäre Phase einzutreten und diese aufrechtzuerhalten, und die Mikroorganismen zur Produktion von rekombinantem Produkt induziert werden.

2. Verfahren nach Anspruch 1, wobei die Mikroorganismen aus der Gruppe *Aspergillus* sp., *Trichoderma* sp., *Mucor* sp., *Pichia* sp., *Fusarium* sp., *Neurospora* sp., *Penicillium* sp., *Streptomyces* sp., *Chrysosporium lucknowense, Mortierella* alpinis ausgewählt sind.

3. Verfahren nach Anspruch 1 oder 2, wobei das Verfahren über einen Zeitraum von 2 - 60 Tagen durchgeführt wird.

4. Verfahren nach einem der Ansprüche 1 bis 3, wobei der Biofilm eine Dicke von 0,1 - 10 mm aufweist.

5. Verfahren nach einem der Ansprüche 1 bis 4, bei dem man ein Induktormolekül der Nährlösung zugibt, so dass die Fließgeschwindigkeit der Nährlösung von der zweiten Seite durch das Substrat und den Biofilm hindurch zu der ersten Seite den Induktor zu dem Mikroorganismus trägt, um dadurch die Produktion wenigstens eines rekombinanten Produkts durch die Mikroorganismen zu induzieren, und wobei es sich bei dem Induktor gegebenenfalls um ein primäres oder sekundäres Stoffwechselprodukt handelt, der sich im Biofilm anreichert, so dass, während der Induktor in der Nährösung durch den Biofilm hindurch getragen wird, dies zu einem Konzentrationsgradienten führt, wobei die Induktorkonzentration näher zum Substrat hin niedriger und weiter vom Substrat entfernt höher ist, um dadurch die Expression wenigstens eines rekombinanten Produkts durch den Mikroorganismus zu induzieren.

6. Verfahren nach Anspruch 7, wobei der Induktor aus wenigstens einem Mitglied der Gruppe Isopropyl-β-D-Thiogalactosid (IPTG) (0,1-1 mM), Methanol (0,5-1,5%) und vom Mikroorganismus produzierte organische Säuren ausgewählt ist.

7. Verfahren nach einem der Ansprüche 1 bis 6, wobei das rekombinante Produkt intrazellulär von den Mikroorganismen produziert wird.

8. Verfahren nach einem der Ansprüche 1 bis 6, wobei das rekombinante Produkt extrazellulär von den Mikroorganismen produziert wird.

9. Verfahren nach einem der Ansprüche 1 bis 8, bei dem man den Biofilm mit einem Gas in Kontakt bringt.

## Revendications

1. Procédé de production d'au moins un produit recombiné dans des conditions aérées, lequel procédé comprend :
la fourniture d'un substrat poreux ayant une première face et une seconde face et qui comprend un film biologique de micro-organismes fixé sur la première face de celui-ci, le substrat étant configuré de façon à permettre le passage d'une solution de nutriment à travers celui-ci et de façon à empêcher le passage de cellules de micro-organismes à travers celui-ci ; et
l'entraînement de l'écoulement d'une solution de nutriment à travers le substrat et le film biologique dans une direction depuis la seconde face de celui-ci jusqu'à la première face de celui-ci dans des conditions aérées, dans lequel le débit de la solution de nutriment depuis la seconde face jusqu'à la première face est de 0,001 à 10 volumes de solution de nutriment par volume de réacteur biologique par heure, de façon à fournir un gradient de nutriment ayant un différentiel de concentration à travers le film biologique, dans lequel la concentration de nutriment est plus élevée à proximité du substrat de façon à entretenir une croissance primaire des micro-organismes et dans lequel la concentration de nutriment est plus faible à une certaine distance du substrat de façon à entraîner l'entrée et le maintien des micro-organismes dans une phase stationnaire, et les micro-organismes étant induits à produire un produit recombiné.

2. Procédé selon la revendication 1, dans lequel les micro-organismes sont choisis dans le groupe constitué de *Aspergillus* sp., *Trichoderma* sp., *Mucor* sp., *Pichia* sp., *Fusarium* sp., *Neurospora* sp., *Penicillium* sp., *Streptomyces* sp., *Chrysosporium lucknowense, Mortierella alpinis.*

3. Procédé selon la revendication 1 ou 2, dans lequel le procédé est réalisé pendant une durée de 2 à 60 jours.

4. Procédé selon l'une quelconque des revendications 1 à 3, dans lequel le film biologique a une épaisseur de 0,1 à 10 mm.

5. Procédé selon l'une quelconque des revendications 1 à 4, qui comprend l'addition d'une molécule d'inducteur à la solution de nutriment de sorte que le débit de la solution de nutriment depuis la seconde face à travers le substrat et le film biologique jusqu'à la première face porte ledit inducteur jusqu'au micro-organisme, de façon à induire la production d'au moins un produit recombiné par les micro-organismes et dans lequel l'inducteur est éventuellement un métabolite primaire ou secondaire qui s'accumule au sein du film biologique, de sorte que l'inducteur soit porté à travers le film biologique dans la solution de nutriment, ce qui conduit à un gradient de concentration, dans lequel la concentration de l'inducteur est plus faible à proximité du substrat et plus élevée à une certaine distance du substrat, de façon à induire l'expression d'au moins un produit recombiné par le micro-organisme.

6. Procédé selon la revendication 5, dans lequel l'inducteur est choisi parmi l'un au moins de l'isopropyl-b-D-thiogalactoside (IPTG) (0,1 à 1 mM), du méthanol (0,5 à 1,5 %) et des acides organiques produits par le micro-organisme.

7. Procédé selon l'une quelconque des revendications 1 à 6, dans lequel le produit recombiné est produit de façon intracellulaire par les micro-organismes.

8. Procédé selon l'une quelconque des revendications 1 à 6, dans lequel le produit recombiné est produit de façon extracellulaire par les micro-organismes.

9. Procédé selon l'une quelconque des revendications 1 à 8, qui comprend la mise en contact du film biologique avec un gaz.
